# EUROPEAN PATENT APPLICATION

(11) **EP 3 124 013 A1**
(43) Date of publication of application: **01.02.2017**
(21) Application number: 15769868.9
(22) Date of filing: 24.03.2015
(51) Int. Cl.: A61K 8/73, A61K 8/55, A61K 8/87, A61Q 1/02

(54) **COSMETIC**

(30) Priority: 28.03.2014 JP 2014068262
(71) Applicant: Kosé Corporation, Chuo-ku, Tokyo 103-8251 (JP)
(72) Inventor: IGARASHI Keiji, Tokyo 114-0005 (JP)
(74) Representative: Hartz, Nikolai
(86) International application number: PCT/JP2015/058828
(87) International publication number: WO 2015/146948

(57) **Abstract**

[Problem]

The present invention has an object to provide a cosmetic which is satisfactorily transferred onto a finger or a small tool, is smoothly spread on the skin, forms a non-sticky and uniform cosmetic film, and can achieve a beautiful finish in which the cosmetic effects last for a prolonged time.

[Means for Resolution]

The cosmetic which overcomes the problem is characterized by comprising components (a) to (c) below: (a) an agar, (b) a hydrophobically modified polyether urethane represented by the following general formula (1):

R¹-{(O-R²)ₖ-OCONH-R³[-NHCOO-(R⁴-O)ₙ-R⁵]ₙ}ₘ, (1)

[wherein, R¹, R² and R⁴ represent hydrocarbon groups which may be the same as or different from one another, R³ represents a hydrocarbon group which may have an urethane bond, R⁵ represents a linear, branched, or secondary hydrocarbon group, m is a numeral value of 2 or more, h is a numeral value of 1 or more, and k and n are each independently a numeral value in a range of 0 to 1000], and (c) a phospholipid.

## Description

### Technical Field

The present invention relates to a cosmetic which can be transferred satisfactorily onto a finger or a small tool, can spread smoothly on application, can achieve a non-sticky, uniform, and beautiful finish, and also is excellent in cosmetic persistence.

In cosmetics, various thickeners have conventionally been used for enhancing use feel and stability and other purposes, and, for example, natural polymers such as xanthan gum and synthetic polymers such as a carboxyvinyl polymer are also used. As one of the synthetic polymers, a hydrophobically modified polyether urethane which has a hydrophilic basal moiety as a backbone and has a hydrophobic moiety at an end and in which the hydrophobic moieties are associated with one another to exhibit a thickening effect in an aqueous medium is known, and, for example, a cosmetic in which a hydrophobically modified polyether urethane and xanthan gum or a carboxyvinyl polymer are used in combination is disclosed (PTL 1). However, the cosmetic has problems of sticky finish, poor cosmetic persistence, and the like.

In a cosmetic using a hydrophobically modified polyether urethane, there is proposed a technique in which a microgel obtained by pulverizing a gel formed using agar, gellan gum, or the like is used in combination with the hydrophobically modified polyether urethane in a specific blending ratio for the purpose of improving viscosity stability in a low to medium viscosity range and touch feel (PTL 2). However, the cosmetic has drawbacks in that the production process is complicated and in addition, the touch feel and the like are peculiar and lacks versatility.

On the other hand, in cosmetics, there is a type in which a surface of the cosmetic is transferred to a small tool such as a puff and a mat to apply the cosmetic to a skin. When transferability of the cosmetic to a small tool and spreadability upon being applied with the small tool are insufficient, non-uniform coverage and the like occurs, failing to provide a beautiful finish, and therefore, it is also important to have characteristics suitable for use with a small tool. Although the present applicant has already proposed a technique for improving the transferring to a small tool and the like by a molding method upon packing (PTL 3), there is also a demand for a means capable of improving transferring to a small tool and the like without being constrained by the production method.

### Citation List

### Patent Literatures

PTL 1: JP-A-2000-239120
PTL 2: JP-B-4979095
PTL 3: JP-A-7-17827

### Summary of Invention

### Technical Problem

The present invention has an object to provide a cosmetic which can be transferred satisfactorily onto a finger or a small tool, can be spread smoothly on the skin, can form a non-sticky and uniform cosmetic film, and can achieve a beautiful finish in which the cosmetic effects last for a prolonged time, that is, which is excellent in cosmetic persistence.

### Solution to Problem

As a result of intensive studies for solving the above problem, the present inventor has found that, by combining an agar with a hydrophobically modified polyether urethane having a specific structure and further using a phospholipid in combination, the resulting cosmetic can be smoothly transferred onto a finger or a small tool, is excellent in spreadability on a skin, can form a beautiful cosmetic film which is non-sticky and uniform without non-uniform coverage, and is excellent also in cosmetic persistence, completing the present invention.

Specifically, the present invention is directed to a cosmetic characterized by comprising components (a) to (c) below:
(a) an agar
(b) a hydrophobically modified polyether urethane represented by the following general formula (1):

   R¹-{(O-R²)ₖ-OCONH-R³[-NHCOO-(R⁴-O)ₙ-R⁵]ₕ}ₘ (1)

   [wherein, R¹, R² and R⁴ represent hydrocarbon groups which may be the same as or different from one another, R³ represents a hydrocarbon group which may have an urethane bond, R⁵ represents a linear, branched, or secondary hydrocarbon group, m is a numeral value of 2 or more, h is a numeral value of 1 or more, and k and n are each independently a numeral value in a range of 0 to 1000], and
(c) a phospholipid.

### Advantageous Effect of Invention

Since the cosmetic of the present invention has spreadability on a skin and forms a non-sticky and uniform cosmetic film, a beautiful finish can be achieved, and also the persistence thereof is excellent. Also when a small tool such as a puff and a mat is used, a certain amount of the cosmetic is quickly deposited uniformly on the small tool only by pressing the surface relatively lightly, and also upon application, the cosmetic spreads smoothly on the skin. Accordingly, a beautifully finishing cosmetic film which is uniform without non-uniform coverage can be formed.

### Brief Description of Drawings

[Fig. 1] Fig. 1 is a plan view of an example of a container for containing the cosmetic of the invention in a state where a lid is removed.
[Fig. 2] Fig. 2 is a partial vertical sectional view of the container of Fig. 1.
[Fig. 3] Fig. 3 shows an example of a puff used when the cosmetic of the invention is applied. In the drawings, (a) is a plan view, (b) is a side view, and (c) is a bottom view.
[Fig. 4] Fig. 4 is stereo images of a cosmetic film obtained by applying the cosmetic of the invention onto a bioskin plate in Test Example 1, captured using a confocal laser scanning microscope. (a) shows a state where the cosmetic is spread and applied on the bioskin plate, and (b) shows a state where further patting three times with a sponge is performed after the application.
[Fig. 5] Fig. 5 is a graph showing load values at the time of pulling a probe pressed on the cosmetic film away, in Test Example 1.

### Description of Embodiments

The component (a), agar, used in the present invention is a known natural polymer and any agar can be used regardless of the production area, the origin, and the like. In addition, as the agar, a so-called low-strength agar in which the molecules are cut by an acid treatment in order to adjust the jelly strength (disclosed in JP-A-5-317008) may be used. The jelly strength of the component (a) is not particularly limited, but preferably 20 to 2000 g/cm², and more preferably 30 to 1000 g/cm². The jelly strength herein means the maximum mass to which a 1 cm² surface of a gel endures for 20 seconds, the gel being obtained by dissolving an agar in water with heat so as to give 1.5% by mass (hereinunder, described only as "%") and allowing the solution to stand at 20°C for 15 hours to solidify the solution (Nikkansui measurement method).

The content of the component (a) agar used in the invention is not particularly limited, but preferably 0.05 to 3% in the whole amount of the cosmetic, and more preferably 0.1 to 2%. In the above range, the gelling power of the agar appropriately functions to result in good transferring onto a small tool, etc. and spreadability on a skin, and a uniform cosmetic film. In addition, when a phospholipid-treated powder or a powder of a component (d) described later is incorporated, dispersion stability of the powder is enhanced, resulting in beautiful finish with suppressed color unevenness, and the cosmetic persistence over time is also enhanced.

The cosmetic of the invention is a cosmetic entirely or partially thickened or gelated, for example, by dissolving or dispersing the component (a) agar in water or another appropriate solvent together with the other raw materials, with heat of about from 60 to 95°C as required and in this state, melt packing the resultant. The cosmetic of the invention does not include a cosmetic in a form in which the agar is previously gelated and then the gel is pulverized and incorporated as a microgel.

The hydrophobically modified polyether urethane (b) used in the invention is an amphiphilic copolymer having a hydrophilic basal moiety as a backbone and having a hydrophobic moiety at an end, and in an aqueous medium, the hydrophobic moieties of the copolymer are associated with one another to exhibit a thickening effect, that is, an associative thickener. The component (b) is represented by the following general formula (1):

R¹-(O-R²)ₖ-OCONH-R³[-NHCOO- (R⁴-O)n-R⁵]ₕ}ₘ (1)

In the above formula (1), R¹, R² and R⁴ represent hydrocarbon groups which may be the same as or different from one another, R³ represents a hydrocarbon group which may have an urethane bond, R⁵ represents a linear, branched, or secondary hydrocarbon group, m is a numeral value of 2 or more, h is a numeral value of 1 or more, and k and n are each independently a numeral value in a range of 0 to 1000. It is suitable that the carbon numbers of R¹, R² and R⁴ are 2 to 4, the carbon number of R³ is 1 to 10, and the carbon number of R⁵ is 8 to 36. m is preferably 2, h is preferably 1, k is preferably 1 to 500, more preferably 100 to 300, and particularly preferably 100 to 150. n is preferably 1 to 200, more preferably 10 to 100, and particularly preferably 10 to 50.

The hydrophobically modified polyether urethane represented by the above formula (1) can be obtained, for example, by allowing one kind or two or more kinds of polyether polyol represented by R¹-[(O-R²)ₖ-OH]ₘ, one kind or two or more kinds of polyisocyanate represented by R³-(NCO)ₕ₊₁, and one kind or two or more kinds of polyether monoalcohol represented by HO-(R⁴-O)ₙ-R⁵ to react with each other.

In this case, R¹ to R^{S} in the general formula (1) are determined depending on the used R¹- [(O-R²)ₖ-OH]ₘ, R³- (NCO)ₕ₊₁, and HO-(R⁴-O)ₙ-R⁵. The ratio of the three components charged is not particularly limited, but it is preferred that the ratio of the hydroxy groups originated in the polyether polyol and the polyether monoalcohol and the isocyanate groups originated in the polyisocyanate meets NCO/OH = 0.8:1 to 1.4:1.

Among them, those with a weight average molecular weight of about 50, 000 (GPC method) which has a structure in which the opposite ends of polyethylene glycol are modified with decyltetradecyl alcohol is suitably used. As a commercial product of such a hydrophobically modified polyether urethane, for example, "ADEKA NOL GT-700" (manufactured by ADEKA CORPORATION) which is PEG-240/HDI copolymer bis decyltetradeceth-20 ether and the like is exemplified. "ADEKA NOL GT-700" corresponds to a compound of the general formula (1) wherein R¹, R², and R⁴ each are a hydrocarbon having 2 carbon atoms, R³ is a hydrocarbon having 6 carbon atoms, R⁵ is a hydrocarbon having 24 carbon atoms, m = 2, h = 1, k = 120, and n = 20.

The content of the hydrophobically modified polyether urethane used in the invention is not particularly limited, but preferably 0.03 to 1.5% in the whole amount of the cosmetic, and more preferably 0.05 to 1%, since the cosmetic is then excellent in the effects of reducing the stickiness in the finish and enhancing the cosmetic persistence.

The mass ratio (b)/(a) to the component (a) in the contents is preferably 0.05 to 15, and more preferably 0.1 to 10. Within the range, the transferring onto a finger or a small tool is smooth and the stickiness in the finish is improved.

As the phospholipid of the component (c), phosphatidylcholine, phosphatidylethanolamine, phosphatidylserine, phosphatidylglycerol, and phosphatidylinositol, sphingophospholipid are exemplified, and the analogues thereof and the compositions containing the same, specifically, soybean lecithin, egg yolk lecithin, corn lecithin, and hydrogenated products thereof are included. Among the phospholipids, soybean lecithin is mentioned as a preferred one, and the hydrogenated product thereof is further preferred. Examples of the commercial products include Lecinol S-10E and Lecinol S-10EZ (both manufactured by Nikko Chemicals Co., Ltd.), HSL-70 (manufactured by YMC CO., LTD), BASIS LS-60HR (manufactured by Nisshin Oillio Group, Ltd.), Lecithin CLO (manufactured by J-OIL MILLS, Inc.), and Egg Yolk Lecithin PL-100P (manufactured by Kewpie Corporation).

As the component (c) phospholipid, a composite in which a lipid membrane reinforcement agent such as cholesterol and phytosterol is mixed in a phospholipid in advance can be used, and examples of the commercial product include PRESOME CS2-101 (Nippon Fine Chemical), PHYTOPRESOME (Nippon Fine Chemical), and PYTOCOMPO-PP (Nippon Fine Chemical).

Furthermore, the component (c) phospholipid may be incorporated in the cosmetic as a phospholipid-treated powder in which a powder is surface treated with a phospholipid in advance. The surface treatment is performed by mixing, heating, baking, a gas phase polymerization process, or the like according to an ordinary method. As the powder to be treated, the same powder as the powder of the component (d) described later can be used. The amount of the phospholipid with which the powder is coated is preferably 0.1 to 8% relative to the powder to be coated, and more preferably 0.5 to 5%.

The content of the component (c) phospholipid used in the invention is not particularly limited, but preferably 0.03 to 3.0% in the whole amount of the cosmetic, and more preferably 0.05 to 2%. Within the range, dispersion stability of the phospholipid-treated powder or the powder in the case of incorporating the component (d) powder described later is enhanced, color unevenness is suppressed, stickiness in the finish is reduced, and furthermore the cosmetic persistence over time is enhanced.

In the cosmetic of the invention, although the component (c) can be incorporated as the above phospholipid-treated powder, or incorporated in a form of the phospholipid alone or an emulsified composition or a composite containing the phospholipid, it is also possible to incorporate both the phospholipid-treated powder and the phospholipid or the emulsified composition or composite containing the phospholipid. By such a combination use, the effects of enhancing the dispersion stability, reducing the stickiness in the finish, and enhancing the cosmetic persistence over time of the phospholipid-treated powder or the powder (d) in the case where the powder (d) is incorporated as described later become more significant.

In the cosmetic of the invention, a powder other than the phospholipid-treated powder is preferably incorporated as the component (d). The powder is incorporated for imparting various cosmetic effects, and any powders used in ordinary cosmetics may be arbitrary used. The powder is not particularly limited in the shape such as a tubular shape, a fusiform shape, and a needle shape, in the particle size such as an atomized form, a fine particle form, and a pigment grade, and in the particle structure such as a porous structure and a non-porous structure, and the like. Inorganic powders, photoluminescent powders, organic powders, coloring powders, composite powders, and the like may be exemplified. Specific examples include inorganic powders such as iron blue, ultramarine blue, red iron oxide, yellow iron oxide, black iron oxide, titanium oxide, zinc oxide, silicic anhydride, aluminum oxide, cerium oxide, magnesium oxide, zirconium oxide, magnesium carbonate, calcium carbonate, chromium oxide, chromium hydroxide, carbon black, aluminum silicate, magnesium silicate, aluminum magnesium silicate, mica, a synthesized mica, a synthesized sericite, sericite, talk, kaolin, silicon carbide, barium sulfate, and boron nitride; photoluminescent powders such as bismuth oxychloride, mica titanium, an iron oxide-treated mica, an iron oxide-treated mica titanium, an organic pigment-treated mica titanium, a (silicon dioxide and titanium oxide)-coated mica, a titanium oxide-treated glass powder, an (iron oxide and titanium oxide)-treated glass powder, and an aluminum powder; organic powders such as magnesium stearate, zinc stearate, N-acyllysine, and nylon; coloring powders such as an organic tar pigment and an organic coloring rake pigment; composite powders such as a fine particle titanium oxide-coated mica titanium, a fine particle zinc oxide-coated mica titanium, a barium sulfate-coated mica titanium, a titanium oxide-containing silicon dioxide, and a zinc oxide-containing silicon dioxide; and silicic anhydride. One kind or two or more kinds thereof may be used. A composite powder in which two or more kinds of the powders are combined may also be used.

In addition, the powder of the component (d) may be a powder whose surface is subjected to a modification treatment with a surface treating agent other than the phospholipid of the component (c). Examples of the treating agent used for hydrophobizing the powder include fluoride compounds, silicone compounds, N-lauroyl-L-lysine, metal soaps, hydrocarbons, higher fatty acids, higher alcohols, esters, waxes, plant/animal waxes, and surfactants, and a complex treatment in which the above materials are combined may be applied. The surface treatment can be conducted by mixing, heating, baking, gas-phase polymerization, or the like according to an ordinary method.

The content of the component (d) is not particularly limited, but preferably 0.1 to 20% in the whole amount of the cosmetic from the viewpoint of imparting various cosmetic effects.

Into the cosmetic of the invention, a water soluble thickener of a component (e) is suitably incorporated. Specific examples include cellulose derivatives such as methylcellulose, hydroxymethylcellulose, hydroxyethylcellulose, hydroxypropylcellulose, and carboxymethylcellulose; natural polysaccharides such as sodium alginate, carrageenan, quince seed gum, pectin, and hyaluronic acid; synthesized polymers such as an acrylic acid/alkyl acrylate copolymer, polyvinyl alcohol, sodium polyacrylate, sodium polymethacrylate, glyceryl polyacrylate, polyvinylpyrrolidone, and gellan gum, and others. Among them, an acrylic acid/alkyl acrylate copolymer, carrageenan, gellan gum, hyaluronic acid, and the like are preferred because of excellent transferring onto a small tool, etc. and cosmetic persistence over time.

The content of the water soluble thickener of the component (e) is preferably 0.001 to 1% in the whole amount of the cosmetic, and more preferably 0.005 to 0.5%. In this range, transferring onto a small tool, etc. is good and cosmetic persistence and the like are also excellent.

The formulation form of the cosmetic of the invention is not particularly limited and any of aqueous, oil-in-water, water-in-oil types may be adopted, but an oil-in-water type is preferred since the effects such as transferability onto a small tool, etc. and cosmetic persistence can then be significantly exhibited.

In cases of oil-in-water formulation forms, as a component constituting the aqueous phase, in addition to water, aqueous components such as polyhydric alcohols or polymers thereof, for example, 1,3-butylene glycol, dipropylene glycol, glycerin, propylene glycol, polypropylene glycol, etc. and sugar alcohols, for example, sorbitol, maltitol, glucose, etc. can be used. The content of the aqueous component is preferably 20 to 95% in the whole amount of the cosmetic from the viewpoint of realizing good transferring and smooth spread on application.

On the other hand, as an oil agent constituting the oil phase, oil agents other than the component (c) phospholipid which are generally used for cosmetics can be used, and regardless of the nature such as solid oils, semisolid oils, liquid oils, and volatile oils and the origin such as animal oils, vegetable oils, and synthetic oils, it is posssible to use hydrocarbons, oils and fats, plant/animal waxes, ester oils, fatty acids, higher alcohols, silicone oils, fluoride oils, lanolin derivatives, oil gellants, and the like. Examples include hydrocarbons such as liquid paraffin, microcrystalline wax, petrolatum, mineral oils, and hydrogenated polyisobutene; fats and oils such as olive oil, jojoba oil, mink oil, and Japan (Rhus Succedanea) wax; plant/animal waxes such as bees wax and candelilla wax; ester oils such as cetyl isooctanoate, isopropyl myristate, isopropyl palmitate, octyldodecyl myristate, neopentyl glycol dioctanoate, cholesterol fatty acid ester, jojoba oil, triglyceride such as glyceryl tri(2-ethylhexanoate) and capric triglyceride, 2-ethylhexyl p-methoxycinnamate, isopropyl palmitate, octyldodecyl myristate, glyceryl trioctanoate, polyglyceryl diisostearate, diglyceryl triisostearate, glyceryl tribehenate, pentaerythrityl rosinate, neopentyl glycol dioctanoate, cholesterol fatty acid ester, and di(cholesteryl behenyl octyldodecyl) N-lauroyl-L-glutamate; fatty acids such as stearic acid and oleic acid; higher alcohols such as cetanol; silicone oils such as methyl polysiloxane, methylphenyl polysiloxane, and a dimethyl polysiloxane polymer having a three dimensional crosslinking structure; and lanoline derivatives such as lanoline fatty acid isopropyl ester and lanoline alcohol. One kind thereof may be used or two or more kinds thereof may be used in combination. The content of the oil agent depends on the formulation form, but is preferably 3 to 50% in the whole amount of the cosmetic, and more preferably 5 to 20%.

In the cosmetic of the invention, in addition to the above components, components used in ordinary cosmetics, for example, a surfactant, a moisturizer, a preservative, an antioxidant, a pH regulator, a metal chelating agent, an ultraviolet absorber, a cosmetic component, and a fragrance may be appropriately incorporated to the extent that does not impair the effects of the invention.

The cosmetic of the invention can be produced by uniformly mixing the essential components (a) to (c) mentioned above, the components (d) to (e) which are used as required, and other optional components according to any known methods. For example, in a case where the components (a) to (d), aqueous components and oily components such as oil agents are used to form an oil-in-water cosmetic, the cosmetic can be produced by a production method comprising the following steps (1) to (5).
(1) Powder materials such as the component (d) are uniformly dispersed and mixed. Here, the component (c) phospholipid may be allowed to function as a dispersant.
(2) The components (a) and (b) are uniformly mixed and dissolved in water, and the mixture of powders obtained in the step (1) is added and mixed.
(3) Oily components such as oil agents are uniformly dissolved with heat. The component (c) may be added here to be allowed to function as an emulsifying dispersant in the next step (4).
(4) Aqueous components such as water are uniformly dissolved with heat, and is then added to the mixture of the oily components obtained in the step (3) and emulsified, and the resultant is cooled with stirring.
(5) The emulsion obtained in the step (4) is added to and mixed in the mixture obtained by the step (2), and the mixture is cooled, thereby obtaining an oil-in-water cosmetic.

The type of the cosmetic of the invention is not particularly limited, and the cosmetic is applicable to an essence, a milky lotion, a base, a liquid-type foundation, a BB Cream, a liquid-type eye color, or the like. The form thereof is also not particularly limited, and any forms such as a solid form, a gel form, a cream form, and a liquid form may be adopted. Among others, solid or gel forms in room temperature are preferred, and in particular, a gel form is suitable since the effects of the invention in smooth transferring, cosmetic persistence over time and the like is then likely to be significantly exhibited. Such a gel cosmetic of the invention has different characteristics than conventional gel cosmetics. That is, the cosmetic is in a gel form which has little fluidity in a usual state, but when the surface is slowly pressed down from the above by a puff, etc. , the surface comes into a liquid form. Accordingly, the cosmetic is deposited uniformly on an application surface of the puff, etc., smoothly spread also on a skin, and then gelated again to form a uniform cosmetic film. Furthermore, when the surface of the cosmetic film is patted with a small tool for finishing (patting), the cosmetic film is spread and additionally applied with the gel form retained, and therefore the surface is gradually smoothened, whereby a uniform and smooth cosmetic film without color unevenness can be obtained. More specifically, a small tool such as a puff is pressed onto a cosmetic surface at a speed of 15 mm/sec or lower, preferably 5 mm/sec or lower to transfer the cosmetic onto the small tool, the transferred cosmetic is applied on a skin, and the small tool is pressed onto the surface of the applied cosmetic at a speed of 20 mm/sec or higher, preferably 30 mm/sec or higher, and then the small tool is pulled away from the cosmetic surface at a speed within the same range, whereby it is possible to form a more uniform and smoother cosmetic film. The lower limit of the speed at which the small tool is pressed on the cosmetic surface when the cosmetic is transferred is approximately 0.1 mm/sec. The speed at which the small tool is pulled away from the cosmetic surface may be the same level as that on pressing. On the other hand, upon patting at finishing, the upper limit of the speed at which the small tool is pressed on the surface of the applied cosmetic is approximately 50 mm/sec. The force at which the small tool is pressed on the cosmetic when transferring or patting the cosmetic is approximately 0.5 to 2 N.

Accordingly, the gel cosmetic of the invention is suitably combined with a container that is suitable for the use method in which the cosmetic is slowly pressed down from the above with a puff having an application surface. A suitable example of such a container is a cosmetic container including a container body for containing the gel cosmetic, an elastic member provided along an inner wall of the container, a mesh member holding a mesh on a holding frame corresponding to the top shape of the container and provided on the surface of the cosmetic, and a lid of the container, wherein the holding frame of the mesh member is placed on the elastic member, the mesh has elasticity, and which is so constituted that when the mesh member is pressed down by the application surface of the puff, the mesh is first sagged, and then a force is transmitted via the mesh holding frame to the elastic member, whereby the elastic member is sagged.

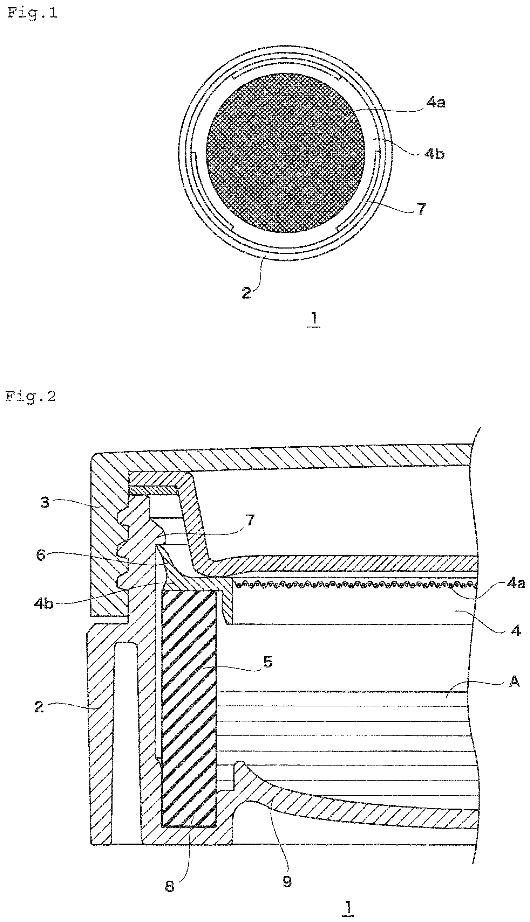

Figs. 1 and 2 show an example of a container suitable for the gel cosmetic of the invention. Fig. 1 is a plan view showing a state where the lid is removed, and Fig. 2 is a partial cross sectional view thereof. In the figures, 1 denotes the container, 2 denotes the container body, 3 denotes the lid, 4 denotes the mesh member, 5 denotes the elastic member, 6 denotes a flange, 7 denotes an locking protrusion, 8 denotes an elastic member receiving portion, 9 denotes a bottom levee structure, and A denotes the gel cosmetic of the invention.

As shown in Fig. 2, the container 1 of the embodiment described above is threaded on an upper outer surface for being screwed with the lid 3, and on an upper inner surface, the locking protrusion 7 for locking the mesh member 4 described later is provided. In addition, on the bottom of the container, the bottom levee structure 9 is formed so as to gradually rise from the center toward the periphery to the position of the elastic member 5, and then, the surface is recessed to form the elastic member receiving portion 8. The elastic member 5 is held on the inner periphery of the container body 2 by the elastic member receiving portion 8 and the mesh member 4 which is locked by the locking protrusion 7.

Fig. 1 shows a shape of the container 1 viewed from the above in a state where the lid 3 is removed, that is, the plane shape thereof. Although the plane shape is a circle in this embodiment, the plane shape of the container is not limited thereto, and may also be an ellipse, a rectangular or a polygonal shape depending on the shape of the puff used. In addition, the container body 2 is preferably in a flat shape where the diameter is larger than the height, and as the material, a material used for a common cosmetic container, for example, plastics, glasses or metals can be used. Incidentally, the bottom levee structure 9 of the container body 2 has not only a function to form the elastic member receiving portion 8 as described above but also a function to make the filled gel cosmetic A easy to be transferred to the last.

The mesh member 4 placed on the top of the gel cosmetic A filled in the container body 2 is composed of a mesh 4a and a holding frame 4b. Among them, the mesh 4a is an elastic fiber, for example, a sheet of approximately from 10 to 100 deniers made of polyester, polyurethane, nylon, rayon, silk, and the like, and the aperture thereof is preferably approximately from 0.3 mm to 5 mm. On the other hand, the holding frame 4b for holding the mesh 4a has substantially the same plane shape as that of the container body 2, and has the mesh 4a attached in the central opening. The holding frame 4b is not connected to the container body 2 and is locked with the locking protrusion 7 of the container body 2 by the flange 6 in the upper portion thereof. The holding frame 4b has a sufficient strength to press down the elastic member 5 describe later.

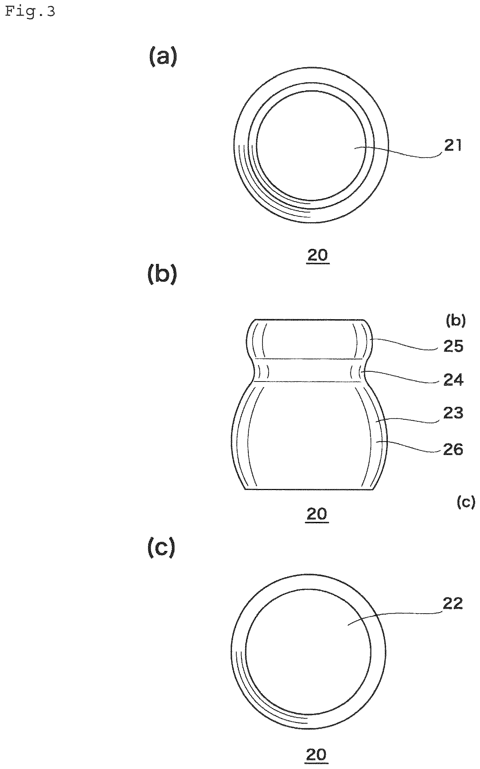

As the puff which can be preferably used for the gel cosmetic of the invention, a sponge puff 20 as shown in Fig. 3 can be exemplified. The puff 20 is formed in an axisymmetric gourd shape having a top surface 21, a bottom surface (application surface) 22 and a side surface 23. On the top surface 21 side of the center of the side surface 23, a constricted part 24 which is constricted so as to have a smaller cross sectional area value than the other parts is formed, and a wider girth part 25 which is bulged toward the expanded radial direction is formed between the constricted part 24 and the top surface 21, and a wider girth part 26 which is bulged toward the expanded radial direction is formed between the constricted part 24 and the bottom surface 22.

The puff 20 has the constricted part 24 having the minimum cross sectional area value between the top surface 21 and the bottom surface 22, the bottom surface 22 is formed with a plain surface which functions as an application surface, a region between the bottom surface 22 and the wider girth part 26 which is the closest to the bottom surface 22 forms an application portion and the bottom surface 22 forms the application surface. By allowing the bottom surface 22 to slowly press the surface of the gel cosmetic A via the mesh 4a, the gel cosmetic can be uniformly transferred onto the puff.

The container for containing the cosmetic of the invention and the small tool for applying the cosmetic are not limited to the above embodiment, and various forms of container and small tool may be used.

### Examples

The present invention is further described below with reference to examples. Incidentally, the present invention is by no means limited by the examples.

Examples 1 to 9 and Comparative Examples 1 to 10: oil-in-water liquid-type foundation

Liquid-type foundations were prepared according to the compositions shown in Tables 1 and 2 and the following production method, and evaluated according to the following evaluation method for <I> smoothness of transferring, <II> absence of color unevenness of the cosmetic film, <III> absence of stickiness in the finish, and <IV> cosmetic persistence over time. The results are shown in Tables 1 and 2 together.

**[Table 1]**

| | | Example | | | Comparative Example | | | | | | |
|---|---|---|---|---|---|---|---|---|---|---|---|
| | Component | 1 | 2 | 3 | 1 | 2 | 3 | 4 | 5 | 6 | 7 |
| 1 | Talc | 3 | 3 | 3 | 3 | 3 | 3 | 3 | 3 | 3 | 3 |
| 2 | Titanium dioxide | 10 | 10 | 10 | 10 | 10 | 10 | 10 | 10 | 10 | 10 |
| 3 | Yellow iron oxide | 2 | 2 | 2 | 2 | 2 | 2 | 2 | 2 | 2 | 2 |
| 4 | Red iron oxide | 0.6 | 0.6 | 0.6 | 0.6 | 0.6 | 0.6 | 0.6 | 0.6 | 0.6 | 0.6 |
| 5 | Black iron oxide | 0.3 | 0.3 | 0.3 | 0.3 | 0.3 | 0.3 | 0.3 | 0.3 | 0.3 | 0.3 |
| 6 | Phospholipid-treated talc *1 | - | - | - | - | - | - | - | - | - | - |
| 7 | Phospholipid-treated titanium oxide *1 | - | - | - | - | - | - | - | - | - | - |
| 8 | Phospholipid-treated yellow iron oxide *1 | - | - | - | - | - | - | - | - | - | - |
| 9 | Phospholipid-treated red iron oxide *1 | - | - | - | - | - | - | - | - | - | - |
| 10 | Phospholipid-treated black iron oxide *1 | - | - | - | - | - | - | - | - | - | - |
| 11 | Silicone-treated talc *2 | - | - | - | - | - | - | - | - | - | - |
| 12 | Silicone-treated titanium oxide *2 | - | - | - | - | - | - | - | - | - | - |
| 13 | Phospholipid | 0.5 | 0.5 | 0.5 | 0.5 | 0.5 | 0.5 | 0.5 | 0.5 | - | - |
| 14 | 1,3-Butylene glycol | 8 | 8 | 8 | 8 | 8 | 8 | 8 | 8 | 8 | 8 |
| 15 | Liquid paraffin | 5 | 5 | 5 | 5 | 5 | 5 | 5 | 5 | 5 | 5 |
| 16 | 2-Ethylhexyl p-methoxycinnamate | 3 | 3 | 3 | 3 | 3 | 3 | 3 | 3 | 3 | 3 |
| 17 | Behenyl alcohol | 0.3 | 0.3 | 0.3 | 0.3 | 0.3 | 0.3 | 0.3 | 0.3 | 0.3 | 0.3 |
| 18 | Phospholipid | 1 | 1 | 1 | 1 | 1 | 1 | 1 | 1 | - | - |
| 19 | Na Stearate | - | - | - | - | - | - | - | - | 1 | - |
| 20 | Sucrose polystearate | - | - | - | - | - | - | - | - | - | 1 |
| 21 | 1,3-Butylene glycol | 5 | 5 | 5 | 5 | 5 | 5 | 5 | 5 | 5 | 5 |
| 22 | Purified water | 20.7 | 20.7 | 20.7 | 20.7 | 20.7 | 20.7 | 20.7 | 20.7 | 20.7 | 20.7 |
| 23 | Fragrance | q.s. | q.s. | q.s. | q.s. | q.s. | q.s. | q.s. | q.s. | q.s. | q.s. |
| 24 | Methyl p-hydroxybenzoate | q.s. | q.s. | q.s. | q.s. | q.s. | q.s. | q.s. | q.s. | q.s. | q.s. |
| 25 | Dipropylene glycol | 10 | 10 | 10 | 10 | 10 | 10 | 10 | 10 | 10 | 10 |
| 26 | Agar*4 | 0.5 | 1 | 0.1 | - | 0.5 | - | - | 0,5 | 0.5 | 0.5 |
| 27 | Hydrophobically modified polyether urethane *5 | 0.5 | 0.1 | 1 | 0.5 | - | 0.5 | 0.5 | - | 0.5 | 0.5 |
| 28 | Carboxyvinyl polymer | - | - | - | - | - | 0.5 | - | 0.5 | - | - |
| 29 | Xanthan gum | - | - | - | - | - | - | 0.5 | - | - | - |
| 30 | Acrylic acid/alkyl acrylate copolymer | 0.25 | 0.25 | 0.25 | 0.25 | 0.25 | 0.25 | 0.25 | 0.25 | 0.25 | 0.25 |
| 31 | Purified water | balance | balance | balance | balance | balance | balance | balance | balance | balance | balance |
| Evaluation item and evaluation result | | | | | | | | | | | |
| I | Smoothness of transferring | ○○ | ○ | ○ | × | | ○ | | | ○ | ○ |
| II | Absence of color unevenness of cosmetic film | ○ | ○○ | ○ | Δ | Δ | ○ | ○ | ○ | ○ | × |
| III | Absence of stickiness in the finish | ○○ | ○ | ○○ | | × | | × | | | × |
| IV | Cosmetic persistence overtime | ○ | ○ | ○○ | | | × | × | | × | × |

**[Table 2]**

| | | Example | | | | | | Comparative Example | | |
|---|---|---|---|---|---|---|---|---|---|---|
| | Component | 4 | 5 | 6 | 7 | 8 | 9 | 8 | 9 | 10 |
| 1 | Talc | - | - | - | - | - | - | - | - | - |
| 2 | Titanium dioxide | - | - | - | - | - | - | - | - | - |
| 3 | Yellow iron oxide | - | - | - | - | - | 2 | - | - | 2 |
| 4 | Red iron oxide | - | - | - | - | - | 0.6 | - | - | 0.6 |
| 5 | Black iron oxide | - | - | - | - | - | 0.3 | - | - | 0.3 |
| 6 | Phospholipid-treated talc *1 | 3 | 3 | 3 | 3 | 3 | - | 3 | 3 | - |
| 7 | Phospholipid-treated titanium oxide *1 | 10 | 10 | 10 | 10 | 10 | - | 10 | 10 | - |
| 8 | Phospholipid-treated yellow iron oxide *1 | 2 | 2 | 2 | 2 | 2 | - | 2 | 2 | - |
| 9 | Phospholipid-treated red iron oxide *1 | 0.6 | 0.6 | 0.6 | 0.6 | 0.6 | - | 0.6 | 0.6 | - |
| 10 | Phospholipid-treated black iron oxide *1 | 0.3 | 0.3 | 0.3 | 0.3 | 0.3 | - | 0.3 | 0.3 | - |
| 11 | Silicone-treated talc *2 | - | - | - | - | - | 3 | - | - | 3 |
| 12 | Silicone-treated titanium oxide *2 | - | - | - | - | - | 10 | - | - | 10 |
| 13 | Phospholipid | - | - | - | - | - | 0.5 | 0.5 | 0.5 | - |
| 14 | 1,3-Butylene glycol | 8 | 8 | 8 | 8 | 8 | 8 | 8 | 8 | 8 |
| 15 | Liquid paraffin | 5 | 5 | 5 | 5 | 5 | 5 | 5 | 5 | 5 |
| 16 | 2-Ethylhexyl p-methoxycinnamate | 3 | 3 | 3 | 3 | 3 | 3 | 3 | 3 | 3 |
| 17 | Behenyl alcohol | 0.3 | 0.3 | 0.3 | 0.3 | 0.3 | 0.3 | 0.3 | 0.3 | 0.3 |
| 18 | Phospholipid | 1 | 1 | 1 | - | - | 1 | 1 | 1 | - |
| 19 | Na Stearate | - | - | - | 1 | - | - | - | - | 1 |
| 20 | Sucrose polystearate | - | - | - | - | 1 | - | - | - | - |
| 21 | 1,3-Butylene glycol | 5 | 5 | 5 | 5 | 5 | 5 | 5 | 5 | 5 |
| 22 | Purified water | 20.7 | 20.7 | 20.7 | 20.7 | 20.7 | 20.7 | 20.7 | 20.7 | 20.7 |
| 23 | Fragrance | q.s. | q.s. | q.s. | q.s. | q.s. | q.s. | q.s. | q.s. | q.s. |
| 24 | Methyl p-hydroxybenzoate | 0.1 | 0.1 | 0.1 | 0.1 | 0.1 | 0.1 | 0.1 | 0.1 | 0.1 |
| 25 | Dipropylene glycol | 10 | 10 | 10 | 10 | 10 | 10 | 10 | 10 | 10 |
| 26 | Agar *4 | 0.5 | 1 | 0.1 | 0.5 | 0.5 | 0.5 | - | 0.5 | 0.5 |
| 27 | Hydrophobically modified polyether urethane *5 | 0.5 | 0.1 | 1 | 0.5 | 0.5 | 0.5 | 0.5 | - | 0.5 |
| 28 | Carboxyvinyl polymer | - | - | - | - | - | - | - | - | - |
| 29 | Xanthan gum | - | - | - | - | - | - | - | - | - |
| 30 | Acrylic acid/alkyl acrylate copolymer | 0.25 | 0.25 | 0.25 | 0.25 | 0.25 | 0.25 | 0.25 | 0.25 | 0.25 |
| 31 | Purified water | balance | balance | balance | balance | balance | balance | balance | balance | balance |
| Evaluation item and evaluation result | | | | | | | | | | |
| I | Smoothness of transferring | ○○ | ○ | ○○ | ○○ | ○○ | ○○ | × | | ○ |
| II | Absence of color unevenness of cosmetic film | ○○ | ○○ | ○○ | ○ | ○ | ○ | ○ | | |
| III | Absence of stickiness in the finish | ○○ | ○○ | ○○ | ○○ | ○○ | ○○ | | | |
| IV | Cosmetic persistence over time | ○○ | ○○ | ○○ | ○ | ○ | ○ | | | × |

*1 Treated with 2% of hydrogenated soy bean phospholipid (PC purity 60 to 85%)
*2 Treated with 2% of dimethicone (20 cs)
*3 Treated with 3% of triethoxycaprylylsilane
*4 Ultra agar AX-100CS (manufactured by Ina Food Industry Co. , Ltd.)
*5 ADEKA NOL GT-700 (manufactured by ADEKA CORPORATION)

### (Production method)

A. The components (1) to (13) are uniformly dispersed by a triple roller.
B. The components (23) to (31) are dissolved with heat while mixing uniformly, and A is added thereto.
C. The components (14) to (20) are uniformly dissolved with heat at 70°C.
D. The components (21) and (22) are uniformly dissolved with heat at 70°C, added to C and emulsified, and cooled with stirring.
E. D is added to B and mixed, and the mixture was cooled and then degassed.
F. E was melted at 80°C, and then filled in a plastic jar with a diameter of 5 cm (see Figs. 1 to 2) at 60°C, whereby an oil-in-water liquid-type foundation was obtained.

### (Evaluation method)

For evaluation items <I> smoothness of transferring, <II> absence of color unevenness of the cosmetic film, <III> absence of stickiness in the finish, and <IV> cosmetic persistence over time, each sample was subjected to a using test by 10 specialized panelists. Each of the panelists evaluated and rated the sample according to the following 7-level evaluation criteria, and the average of the scores of all the panelists was calculated to make judgement in 4 levels according to the following determination criteria. Incidentally, as for <I> smoothness of transferring, a sample filled in the jar (see Figs. 1 to 2) was lightly pressed from the above of a nylon mesh with a cylindrical puff with a diameter of 3 cm (see Fig. 3) and the transferring onto the puff was evaluated. In addition, as for <IV> cosmetic persistence over time, the degree of persistence of the cosmetic film at 8 hours after application of the sample was evaluated.

### (Evaluation criteria)

| (Score) : | (Evaluation) |
|---|---|
| 6 : | Very good |
| 5 : | Good |
| 4 : | Slightly good |
| 3 : | Normal |
| 2 : | Slightly poor |
| 1 : | Poor |
| 0 : | Very poor |

### (Determination criteria)

| (Average of scores): | (Determination) |
|---|---|
| 5.0 or higher: | ○○ (Very good) |
| 3.5 or higher and lower than 5.0: | ○ (Good) |
| | |
| 1.5 or higher and lower than 3.5: | Δ (Normal) |
| | |
| Lower than 1.5: | × (No good) |

### (Results)

As is apparent from the results in Table 1, in the oil-in-water liquid-type foundation of the invention in which (a) an agar, (b) a hydrophobically modified polyether urethane, and (c) a phospholipid are used, by filling the foundation after heating again, while the component (a) agar uniformly forms gel, moderate collapsibility is imparted to the gel by combined with the component (b) hydrophobically modified polyether urethane, whereby the oil-in-water liquid-type foundation is excellent in all the items of <I> smoothness of transferring, <II> absence of color unevenness of the cosmetic film, <III> absence of stickiness in the finish, and <IV> cosmetic persistence over time. The same was true when the content ratio of the components (a) and (b) was changed (Examples 1 to 3).

In contrast, Comparative Example 1 in which the component (a) agar was not incorporated was poor the transferring onto a puff, and also poor in the dispersion stability of the powder to make color unevenness of the cosmetic film significant, and even when the agar was replaced with a carboxyvinyl polymer or xanthan gum, the sample was significantly poor in terms of reducing the stickiness in the finish and in the cosmetic persistence (Comparative Examples 3 and 4). Comparative Example 2 which lacks the component (b) hydrophobically modified polyether urethane was poor in the transferring onto a puff, and particularly poor in terms of reducing the stickiness in the finish, and even when the hydrophobically modified polyether urethane was replaced with a carboxyvinyl polymer, the sample was poor in terms of reducing the stickiness, in the cosmetic persistence, and the like (Comparative Example 5). Furthermore, Comparative Examples 6 and 7 in which the component (c) phospholipid was replaced with sodium stearate or sucrose polystearate were insufficient in terms of reducing the stickiness, in the cosmetic persistence, and the like.

From the results of Table 2, Examples 4 to 6 further using a phospholipid-treated powder are highly excellent in all the items. On the other hand, even if the phospholipid-treated powder was incorporated, Comparative Examples 8 and 9 which lack the component (a) or (b) were insufficient in terms of the transferring onto a puff and in the stickiness. In addition, even when a silicone-treated powder was used in place of the phospholipid-treated powder, the sample was poor in suppressing the color unevenness and in the cosmetic persistence (Comparative Example 10).

### Example 10: oil-in-water base

An oil-in-water bases was prepared according to the composition and production method shown below.

| (Composition) | (%) |
|---|---|
| (1) Lecithin-treated titanium oxide | 4.0 |
| (2) Lecithin-treated red iron oxide | 0.08 |
| (3) Lecithin-treated yellow iron oxide | 0.40 |
| (4) Lecithin-treated black iron oxide | 0.04 |
| (5) Lecithin-treated talc | 1.0 |
| (6) 1,3-Butylene glycol | 2.0 |
| (7) Sorbitan sesquioleate | 0.2 |
| (8) Mineral oil | 1.4 |
| (9) Glyceryl tris(2-ethylhexanoate) | 0.6 |
| (10) Ethylhexyl methoxycinnamate | 2.0 |
| (11) Triethanolamine stearate | 1.0 |
| (12) Cetostearyl alcohol | 0.5 |
| (13) Purified water | 3.0 |
| (14) Methyl p-hydroxybenzoate | q.s. |
| (15) Agar | 2.0 |
| (16) Glycerin | 0.5 |
| (17) 1,3-Butylene glycol | 10.0 |
| (18) Hydrophobically modified polyether urethane | 0.3 |
| (19) Purified water | balance |

### (Production method)

A. The components (14) to (19) are uniformly mixed and dissolved at 90°C.
B. The components (1) to (7) are uniformly dispersed by a triple roller and added to A with stirring.
C. The components (8) to (12) are uniformly dissolved with heat at 75°C.
D. The component (13) is heated to 75°C, and added to C with stirring by a HOMO DISPER (model 2.5, manufactured by PRIMIX Corporation).
E. D cooled with stirring was added to B with stirring.
F. The mixture was cooled and then degassed, again dissolved with heat at 75°C, and filled in a tube container equipped with an application unit at 55°C, whereby an oil-in-water base was obtained.

The oil-in-water base of Example 10 obtained above was good in dispersion stability of powder and had no color unevenness, and very good in the smoothness of transferring, the absence of stickiness in the finish, and the cosmetic persistence over time.

### Test Example 1

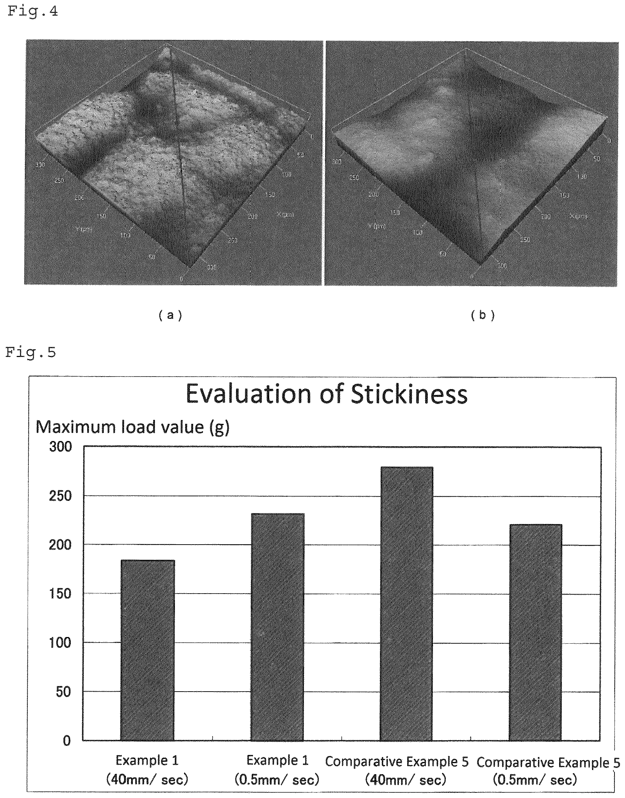

The cosmetic of Example 1 was filled in a jar (see Figs. 1 to 2), a cylindrical puff (see Fig. 3) with a diameter of 3 cm was allowed to slowly press in a manner like stumping three times from the above of a nylon mesh to transfer the cosmetic onto the puff, the cosmetic was spread and applied on a bioskin plate No. 10A (manufactured by Beaulax Co. , Ltd.), and then the surface of the applied cosmetic was patted three times with the same puff. The states of the cosmetic surface before and after the patting were captured by a confocal laser scanning microscope LSM-700 (manufactured by ZEISS). The results are shown in Fig. 4.

The cosmetic of Example 1 or Comparative Example 5 was applied on an artificial leather (SUPPLALE: manufactured by Idemitsu Technofine Co., Ltd.) to form a coating film of 200 µm thickness. With a texture analyzer (manufactured by EKO Instruments), a cylinder shaped probe (2.5 cm diameter) was pressed on the coating film with a force of 20 g for 5 seconds, and when the probe was pulled away at a speed of 40 mm/sec or a speed of 0.5 mm/sec, the load value (g) was measured. The results (the maximum load values) are shown in Fig. 5.

It is recognized from Fig. 4 that, as compared to the state (a) where the cosmetic of Example 1 is spread and applied, in the state (b) after further patting three times with a sponge, the surface of the coating film is more highly smoothened, and that, also in actual use, fine unevenness in the skin is more concealed and a more uniform cosmetic film is formed. The reason for this is as follows: the cosmetic of the invention has a characteristic in that, as the speed of pulling a small tool away from a cosmetic surface increases, the tackiness decreases. The magnitude of the value in Fig. 5 is proportional to the magnitude of the tackiness between the small tool and the cosmetic surface. In Comparative Example 5, as the speed of pulling away from the cosmetic increases, the load value increases, whereas in Example 1, as the speed of pulling away from the cosmetic increases, there is a tendency to show a lower load value. This shows that when a small tool is slowly pressed on a cosmetic surface, the cosmetic is transferred onto the small tool, and when the patting is performed in finishing, since the speed of pulling away of the small tool from the surface of the applied cosmetic is higher, the coating surface can be more highly smoothened without stickiness.

### Industrial Applicability

The cosmetic of the invention is satisfactorily transferred onto a finger or a small tool, is smoothly spread on a skin, forms a non-sticky and uniform cosmetic film, can achieve a beautiful finish, and is excellent in cosmetic persistence. Accordingly, the cosmetic can be used suitably as a makeup cosmetic and the like.

**Reference Signs List**

| | |
|---|---|
| 1 | Container |
| 2 | Container body |
| 3 | Lid |
| 4 | Mesh member |
| 4a | Mesh |
| 4b | Holding frame |
| 5 | Elastic member |
| 6 | Flange |
| 7 | Locking protrusion |
| 8 | Elastic member receiving portion |
| 9 | Bottom levee structure |
| A | Gel cosmetic |
| 20 | Puff |
| 21 | Top surface |
| 22 | Bottom surface |
| 24 | Constricted part |
| 25 | Upper wider girth part |
| 26 | Lower wider girth part |

## Claims

1. A cosmetic comprising components (a) to (c) below:
(a) an agar;
(b) a hydrophobically modified polyether urethane represented by the following general formula (1):
R¹-{(O-R²)ₖ-OCONH-R³[-NHCOO- (R⁴-O)ₙ-R⁵]ₕ}ₘ (1)
[wherein, R¹, R² and R⁴ represent hydrocarbon groups which may be the same as or different from one another, R³ represents a hydrocarbon group which may have an urethane bond, R⁵ represents a linear, branched, or secondary hydrocarbon group, m is a numeral value of 2 or more, h is a numeral value of 1 or more, and k and n are each independently a numeral value in a range of 0 to 1000], and
(c) a phospholipid.

2. The cosmetic according to claim 1, wherein the mass ratio (b) / (a) of the contents of the component (b) and the component (a) is 0.05 to 15.

3. The cosmetic according to claim 1 or 2, wherein the content of the component (a) is 0.05 to 3% by mass.

4. The cosmetic according to any one of claims 1 to 3 , wherein the content of the component (b) is 0.03 to 1.5% by mass.

5. The cosmetic according to any one of claims 1 to 4, wherein the content of the component (c) is 0.03 to 3% by mass.

6. The cosmetic according to any one of claims 1 to 5, further having a powder incorporated as a component (d).

7. The cosmetic according to any one of claims 1 to 6, which is of an oil-in-water type.

8. The cosmetic according to any one of claims 1 to 7, which is in a gel form.

9. A cosmetic method comprising transferring the cosmetic as set forth in claim 8 onto a small tool and applying the transferred cosmetic on a skin, wherein the cosmetic is transferred by pressing the small tool onto a surface of the cosmetic at a speed of 15 mm/sec or lower, the transferred cosmetic is applied on the skin, subsequently the small tool is pressed onto a surface of the applied cosmetic and then the small tool is pulled away from the surface of the cosmetic, at speeds of 20 mm/sec or higher.
